# EUROPÄISCHE PATENTSCHRIFT

(11) **EP 0 925 775 B1**
(45) Veröffentlichungstag und Bekanntmachung des Hinweises auf die Patenterteilung: **07.05.2003**
(21) Anmeldenummer: 98121895.1
(22) Anmeldetag: 18.11.1998
(51) Int. Cl.: A61K 7/06, A61K 7/50

(54) **Haarbehandlungsmittel**
Hair treatment agent
Agent de traitement du cheveu

(30) Priorität: 21.11.1997 DE 19751589
(43) Veröffentlichungstag der Anmeldung: 30.06.1999
(73) Patentinhaber: KPSS-Kao Professional Salon Services GmbH, 64297 Darmstadt (DE)
(72) Erfinder: Grit, Mustafa, Dr., 64579 Gernsheim (DE)

(56) Entgegenhaltungen:
- SE-C- 500 222
- DATABASE CHEMICAL ABSTRACTS [Online] STN Zusammenfassung 124: 90 992, XP002136364 & JP 07 018578 A (LION CORP.) 20. Januar 1995 (1995-01-20)

## Beschreibung

Die vorliegende Erfindung betrifft die Verwendung eines Mittels zur Haarbehandlung, insbesondere zur Haarkonditionierung, dessen Verwendung dem menschlichen Haar vor allem ein verbessertes Volumen, erhöhten Glanz und leichte Naß- und Trockenkämmbarkeit verleiht sowie auch in der Lage ist, geschädigtes Haar zu regenerieren.

Mittel zum Konditionieren von menschlichen Haaren sind seit langem bekannt.
Sie enthalten in der Regel quaternäre Ammoniumverbindungen, die mindestens eine langkettige Alkyl- oder Alkenylgruppe aufweisen, und gegebenenfalls auch Polymere.
Solche Mittel werden üblicherweise als wäßrige Dispersionen bzw. Emulsionen, Mikroemulsionen, Gele oder auch in Aerosolform konfektioniert und als Haarspülungen, Kuren etc. eingesetzt.
Eine Übersicht über die bekannten Haarnachbehandlungsmittel und ihre Zusammensetzung findet sich in der Monographie von K. Schrader, Grundlagen und Rezepturen der Kosmetika, 2. Aufl. (1989), S. 722 bis 781, insbesondere S. 728 bis 737.
Diese bekannten Zusammensetzungen sind aber noch verbesserungsfähig.

Es wurde nunmehr gefunden, daß ein Haarbehandlungsmittel, das dem Haar bei topischer Anwendung verbesserte Eigenschaften insbesondere einen deutlich erhöhten Volumeneffekt, erhöhten Glanz sowie eine leichtere Kämmbarkeit verleiht, und auch eine Regenerierung von vorgeschädigtem Haar bewirken kann, dann erhalten wird, wenn man einem solchen Mittel auf wäßriger Basis eine Kombination aus
a) mindestens einer Fettsäure mit 10 bis 24 Kohlenstoffatomen; und
b) mindestens einer Verbindung der allgemeinen Formel I 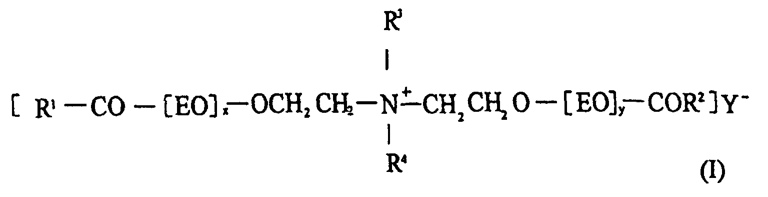 in der R¹ und R² für eine gegebenenfalls hydroxysubstituierte C₈-C₂₂-Alkyl- oder Alkenylgruppe, R³ und R⁴ für eine C₁-C₃-Alkylgruppe oder eine Gruppe -CH₂-CH₂-O-[EO]_{z}H sowie x,y und z für 0 bis 5 und Y⁻ für ein Anion stehen zugibt.

Als Fettsäuren in den erfindungsgemäßen Zusammensetzungen werden bevorzugt solche mit 14 bis 22 Kohlenstoffatomen in einer Menge von etwa 0,1 bis 10 Gew.-%, insbesondere 0,5 bis 8 Gew.-%, bezogen auf die Gesamtzusammensetzung, verwendet. Besonders geeignet sind Behensäure und Stearinsäure; jedoch können auch andere Fettsäuren wie beispielsweise Myristinsäure, Palmitinsäure oder Ölsäure oder auch Gemische natürlicher oder synthetischer Fettsäuren wie Kokosfettsäure eingesetzt werden.

Eine besonders bevorzugte Verbindung der Formel I ist im Rahmen der Erfindung eine solche, in der die Reste R¹ und R² jeweils eine Oleylgruppe oder eine C₁₂-C₁₈-Alkylgruppe, der Rest R³ eine Methylgruppe und der Rest R⁴ eine Gruppe -CH₂-CH₂-O-[EO]_{z}-H bedeuten.

Das Anion Y- ist vorzugsweise ein Halogenid wie Cl⁻ oder Br⁻, ein niederes Alkylsulfat, z.B. Methosulfat, oder ein Alkylphosphat, jedoch können selbstverständlich auch andere Reste eingesetzt werden.

Der bevorzugte Anteil an Verbindungen der Formel I in den erfindungsgemäßen Haarbehandlungsmitteln liegt bei 0,1 bis 20, vorzugsweise 0,5 bis 15, insbesondere 1 bis 10 Gew.-%, berechnet auf die Gesamtzusammensetzung des Mittels.
Diese Verbindungen sind an sich bekannt und beispielsweise unter den Handelsnamen "Schercoquat^{A}", "Dehyquart^{R}F30" und "Tetranyl^{R}" im Handel.

Der Einsatz dieser Verbindungen, sogenannter "Esterquats", in Haarpflegemitteln ist an sich bekannt und beispielsweise in der WO-A 93/10748, der WO-A 92/06899 und der WO-A 94/16677 beschrieben, wo sich jedoch keinerlei Hinweise auf die erfindungsgemäße Kombination und deren vorteilhafte Eigenschaften finden.

Das Haarbehandlungsmittel kann als weiteren bevorzugten Bestandteil noch mindestens eine Verbindung, ausgewählt aus der Gruppe 1-Methoxypropanol(-2), 1-Ethoxypropanol(-2), Diethylenglykolmonomethyl- oder -ethylether, Dipropylenglykolmonomethyl- oder -ethylether, Benzylalkohol, Benzyloxyethanol, Phenylethylalkohol, Phenoxyethanol und/oder Zimtalkohol, vorzugsweise in einer Menge von 0,5 bis 25, insbesondere 1 bis 20, vor allem 2,5 bis 15 Gew.-%, berechnet auf die Geamtzusammensetzung des Mittels, enthalten. Bevorzugte Verbindungen aus dieser Gruppe sind Ethoxydiglykol und Benzyloxyethanol.

Die Haarbehandlungsmittel können natürlich zusätzlich die in solchen Mitteln üblichen Bestandteile enthalten; es wird, zur Vermeidung von Wiederholungen, wiederum auf K. Schrader, "Grundlagen und Rezepturen der Kosmetika", 2. Aufl. (1989), S. 722-771, verwiesen.

Geeignete Zusatzstoffe sind beispielsweise synthetische oder natürliche haarkonditionierende Polymere, vorzugsweise in einer Menge von 0,1 bis 2,5, insbesondere 0,25 bis 1,5 Gew.-% der Gesamtzusammensetzung.

Besonders bevorzugt sind hierbei die unter der CTFA-Bezeichnung "Polyquaternium" bekannten kationischen (Co-)Polymeren, alleine oder auch im Gemisch mit nichtionischen, anionischen und/oder amphoteren Polymeren, beispielsweise solchen vom Typ "Amphomer^{R}".

Auch die als Konditioniermittel seit langem bekannten, eine oder zwei langkettige, insbesondere C₁₀-C₂₂-Alkylreste enthaltenden quaternären Ammoniumverbindungen können mitverwendet werden.
Geeignete langkettige quaternäre Ammoniumverbindungen, die allein oder im Gemisch miteinander eingesetzt werden können, sind insbesondere Cetyltrimethylammoniumchlorid, Dimethyldicetylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyl- dihydriertes-Talgammoniumchlorid, Laurylpyridiniumchlorid, Lauryldimethylbenzylammoniumchlorid, Behenyltrimethylammoniumchlorid Lauryltrimethylammoniumchlorid, Tris-(oligooxyethyl)alkylammoniumphosphat, Cetylpyridiniumchlorid, etc. Gut geeignet sind auch die in der EP-A 472 107 geoffenbarten quaternären Ammoniumsalze.
Im Prinzip sind alle quaternären Ammoniumverbindungen, wie sie im jeweils gültigen "CTFA International Cosmetic Ingredient Dictionary" unter dem Trivialnamen "Quaternium" aufgeführt sind, einsetzbar.
Ihr Anteil liegt vorzugsweise bei etwa 0,1 bis 10, insbesondere etwa 0,5 bis 7,5 Gew.-%, berechnet auf die Gesamtzusammensetzung.

Ein weiterer besonders geeigneter Zusatzstoff ist ein Ceramid, insbesondere der Art, wie es aus der EP 227 994 A1 und der WO-A 96/37462 bekannt ist, jedoch sind auch andere Ceramide, beispielsweise die aus der WO-A 97/15724 oder der EP 647 617 B1 bekannten Ceramide, geeignet.
Deren Menge in den Haarbehandlungsmitteln liegt zweckmäßigerweise bei etwa 0,01 bis 10, vorzugsweise etwa 0,05 bis 7,5, insbesondere etwa 0,1 bis 5 Gew.-%, berechnet auf die Gesamtzusammensetzung.

Weitere Zusatzstoffe, deren Art und Menge natürlich von der Applikationsform des Mittels abhängig sind, sind Fette, Fettalkohole, Emulgatoren, pH-Regulatoren, Lösungs- und Verdünnungsmittel, Lösungsvermittler, Konservierungsmittel, Parfums, etc.

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven-bzw. Sojaöl, Lanolin und dessen Derivate, ebenso Mineralöle wie Paraffinöl und Vaseline.

Synthetische Öle und Wachse sind beispielsweise Silikonöle, Polyethylengykole, etc. Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethlenglykol- und Polyglycerylfettsäureester wie PEG-7-glycerylcocoat, Cetylpalmitat, etc.

Diese hydrophoben Komponenten sind in der erfindungsgemäßen Zusammensetzung vorzugsweise in einer Gesamtmenge von etwa 0,5 bis etwa 10, insbesondere etwa 1 bis 7,5, vor allem etwa 1,5 bis 5 Gew.-%, berechnet auf die Gesamtzusammensetzung, enthalten.

Ebenso können neben den oben erwähnten quaternären langkettigen Ammoniumverbindungen auch andere oberflächenaktive Stoffe, insbesondere amphotere bzw zwitterionische und/oder nichtionische Tenside, deren einschlägige Verwendung natürlich an sich bekannt ist, eingesetzt werden.
Eine beispielhafte Zusammenfassung der Herstellung solcher Mittel findet sich ebenfalls in der bereits erwähnten Monographie von K. Schrader, S. 798 bis 818, insbesondere S. 804 ff.

Eine bevorzugte Tensidgruppe sind dabei die bekannten C₈-C₂₀-Alkylpolyglucoside, vorzugsweise solche mit einem Polymerisationsgrad von etwa 1,1 bis etwa 5, in einer bevorzugten Menge von etwa 0,5 bis etwa 20, insbesondere 1 bis etwa 10 Gew.-%, berechnet auf die Gesamtzusammensetzung.

Die haarkonditionierenden Mittel liegen vorzugsweise als, wäßrige oder wäßrig/alkoholische Lösung, wäßrige Emulsion, Mikroemulsion, Dispersion oder opakes oder transparentes Gel vor, und können auch als Aerosole konfektioniert werden. Solche Zusammensetzungen und ihre Herstellung sind dem Fachmann grundsätzlich bekannt und bedürfen daher keiner näheren Erläuterung.

Der pH-Wert der Haarbehandlungsmittel ist nicht kritisch; er kann vorzugsweise bei 3 bis etwa 8, insbesondere zwischen 4 und 6,5, liegen.

Die folgenden Beispiele illustrieren die Erfindung

### Beispiel 1

| | |
|---|---|
| Behensäure | 1,0 (Gew.-%) |
| Avocadin^{R} | 0,5 |
| Verbindung der Formel I (R¹=R²=Oleyl; R³=CH₃; R⁴=CH₂-CH₂-OH; Y=CH₃SO₄⁻) | 1,0 |
| Steartrimoniumchlorid | 1,0 |
| 1,2-Propandiol | 5,0 |
| Cocoamidopropylbetain | 1,5 |
| C₁₂-C₂₄-Alkylpolyglucosid (P.D.∼1,5) | 2,5 |
| Benzyloxyetbanol | 5,0 |
| Konservierungsmittel | 0,3 |
| Parfum | 0,3 |
| Wasser | ad 100,0 |

### Beispiel 2

| | |
|---|---|
| Behensäure | 2,0 (Gew.-%) |
| Stearinsäure | 1,0 |
| N-(3-Hexadecyloxy-2-hydroxypropyl)-N-2-hydroxyethyldecanamid (Ceramid) | 0,5 |
| Verbindung der Formel I (R¹=C₁₈H₃₇-Alkyl; R²= C₁₁H₂₃ -Alkyl; R³=R⁴=CH₃; Y=Cl⁻) | 3,0 |
| Steartrimoniumchlorid | 1,0 |
| 1,2-Propandiol | 2,5 |
| Benzyloxyethanol | 5,0 |
| C₈-C₂₀-Alkylpolyglucosid (P.D.∼1,4) | 4,5 |
| Parfum | 0,3 |
| Konservierungsmittel | 0,3 |
| Wasser | ad 100,0 |

### Beispiel 3

| | |
|---|---|
| Behensäure | 2,5 (Gew.-%) |
| Avocadin^{R} | 0,5 |
| Verbindung der Formel I (R¹=R²=C₁₂H₃₁; R³=CH₃; R⁴=CH₂-CH₂-OH; Y = Cl⁻) | 3,5 |
| 1,2-Propandiol | 5,0 |
| Benzyloxyethanol | 5,0 |
| C₁₂-C₁₄-Alkylpolyglucosid (P.D.∼1,5) | 3,5 |
| Parfum | 0,3 |
| Konservierungsmittel | 0,4 |
| Wasser | ad 100,0 |

Mit den erfindungsgemäß zusammengesetzten Produkten wurde sowohl mit als auch ohne Ausspülen nach 10-minütiger Einwirkung eine exzellente haardkonditionierende Wirkung und ein Glanz erreicht, die deutlich besser waren als beim Weglassen der Komponente nach Formel I bzw. der Fettsäuren (Behensäure, Stearinsäure).
Auch die Naß- und Trockenkämmbarkeit des behandelten Haares war deutlich verbessert. Diese Effekte konnten auch durch in vitro-Untersuchungen bestätigt werden.

## Patentansprüche

1. Verwendung eines Mittels zur Haarbehandlung auf wäßriger Grundlage, enthaltend ein Gemisch aus
a) mindestens einer C₁₀-C₂₄-Fettsäure, und
b) mindestens einer Verbindung der allgemeinen Formel I 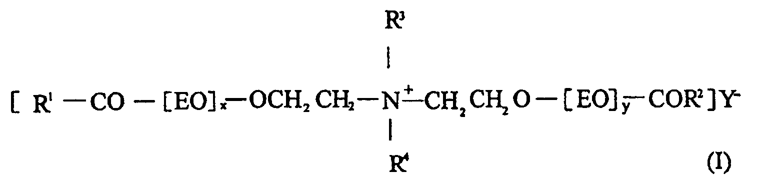 in der R¹ und R² jeweils für eine gegebenenfalls hydroxysubstituierte C₈-C₂₂- Alkyl- oder Alkenylgruppe, R³ und R⁴ für eine C₁-C₃-Alkylgruppe oder eine Gruppe -CH₂-CH₂-O-[EO]_{z}-H, sowie x,y und z für 0 bis 5 und Y⁻ für ein Anion stehen.

2. Verwendung eines Mittels zur Haarbehandlung nach Anspruch 1, enthaltend 0,1 bis 10 Gew.-% mindestens einer C₁₀-C₂₄-Fettsäure, berechnet auf die Gesamtzusammensetzung.

3. Verwendung eines Mittels zur Haarbehandlung nach Anspruch 1 oder 2, enthaltend 0,1 bis 20 Gew.-% mindestens einer Verbindung der Formel I, berechnet auf die Gesamtzusammensetzung.

4. Verwendung eines Mittels zur Haarbehandlung nach einem oder mehreren der Ansprüche 1 bis 3, **dadurch gekennzeichnet, daß** die Reste R¹ und R² jeweils einen Oleylrest, der Rest R³ eine Methylgruppe, der Rest R⁴ eine Gruppe -CH₂-CH₂-OH, und x und y 0 bedeuten.

5. Verwendung eines Mittels zur Haarbehandlung nach einem oder mehreren der Ansprüche 1 bis 3, **dadurch gekennzeichnet, daß** die Reste R¹ und R² jeweils eine C₁₂-C₁₈-Alkylgruppe, der Rest R³ eine Methylgruppe, und der Rest R⁴ eine Gruppe -CH₂-CH₂-O-[EO]_{z}H und x, y und z 0 bedeuten.

6. Verwendung eines Mittels zur Haarbehandlung nach einem oder mehreren der Ansprüche 1 bis 5, enthaltend Behensäure.

7. Verwendung eines Mittels zur Haarbehandlung nach einem oder mehreren der Ansprüche 1 bis 6, enthaltend zusätzlich mindestens eine Verbindung, ausgewählt aus der Gruppe 1-Methoxypropanol(-2), 1-Ethoxypropanol(-2), Diethylenglykolmonomethyl- oder -ethylether, Dipropylenglykolmonomethyl- oder -ethylether, Benzylalkohol, Benzyloxyethanol, Phenylethylalkohol, Phenoxyethanol und/oder Zimtalkohol.

8. Verwendung eines Mittels zur Haarbehandlung nach einem oder mehreren der Ansprüche 1 bis 7, enthaltend zusätzlich mindestens ein quaternäres Ammoniumsalz mit ein oder zwei C₁₀-C₂₂-Alkylgruppen.

9. Verwendung eines Mittels zur Haarbehandlung nach einem oder mehreren der Ansprüche 1 bis 8, enthaltend zusätzlich mindestens ein amphoteres bzw. zwitterionisches und/oder nichtionisches Tensid.

10. Verwendung eines Mittels zur Haarbehandlung nach Anspruch 9, enthaltend als nichtionisches Tensid ein C₈-C₂₀-Alkylpolyglucosid.

## Claims

1. Use of a composition for the treatment of human hair on aqueous carrier basis, comprising a mixture of
a) at least one C₁₀-C₂₄-fatty acid, and
b) at least one compound of the general formula (I) 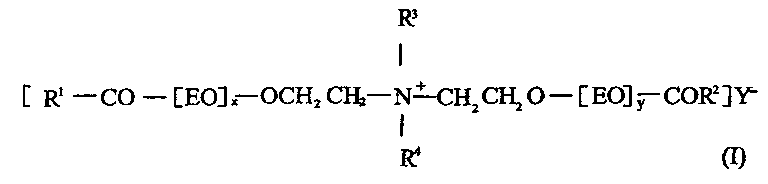
wherein R¹ and R² are each an optionally hydroxy-substituted C₈-C₂₂-alkyl or alkenyl group, R³ and R⁴ are C₁-C₃-alkyl group or a group CH₂-CH₂-O-[EO]_{z}H, x, y and z are 0 to 5, and Y⁻ is an anion.

2. Use of a composition for the treatment of human hair according to claim 1, comprising 0.1% to 10% by weight of at least one C₁₀-C₂₄-fatty acid, calculated to the total composition.

3. Use of a composition for the treatment of human hair according to claim 1 or 2, comprising 0.1% to 20% by weight of at least one compound of formula (I), calculated to the total composition.

4. Use of a composition for the treatment of human hair according to one or more of claims 1 to 3, wherein the groups R¹ and R² each are an oleyl group, R³ is a methyl group, R⁴ is a group -CH₂-CH₂-OH, and x and y are 0.

5. Use of a composition for the treatment of human hair according to one ore more of claims 1 to 3, wherein the groups R¹ and R² each are a C₁₂-C₁₈-alkyl group , R³ is a methyl group, R⁴ is a group -CH₂-CH₂-O-[EO]_{z}H, and x, y and z are 0.

6. Use of a composition for the treatment of human hair according to one ore more of claims 1 to 5, comprising behenic acid.

7. Use of a composition for the treatment of human hair according to one ore more of claims 1 to 6, comprising additionally at least one compound, selected from the group 1-methoxypropanol(-), 1-ethoxypropanol(-2), diethyleneglycol monomethyl or -ethyl ether, dipropyleneglycol monomethyl or -ethyl ether, benzyl alcohol, benzyloxyethanol, phenylethyl alcohol, phenoxyethanol and/or cinnamyl alcohol.

8. Use of a composition for the treatment of human hair according to one or more of claims 1 to 7, comprising additionally at least one quaternary ammonium salt with at least one or two C₁₀-C₂₂-alkyl groups.

9. Use of a composition for the treatment of human hair according to one or more of claims 1 to 8, comprising additionally at least one amphoteric or zwitterionic and/or non-ionic surfactant.

10. Use of a composition for the treatment of human hair according to one or more of claims 1 to 9, comprising as non-ionic surfactant a C₈-C₂₀-alkyl polyglucoside.

## Revendications

1. Utilisation d'un agent pour le traitement des cheveux sur une base aqueuse, contenant un mélange constitué
a) d'au moins un acide gras C₁₀-C₂₄, et
b) d'au moins une composition de la formule générale I 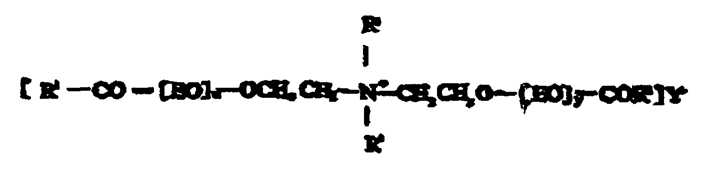 où R¹ et R² représentent respectivement un groupement alkyle ou alkényle C₈-C₂₂ éventuellement hydroxysubstitué, R³ et R⁴ représentent un groupement alkyle C₁-C₃ ou un groupement -CH₂-CH₂-O-[EO]₂-H₂ ,et où x, y et z représentent 0 à 5 et Y représente un anion.

2. Utilisation d'un agent pour le traitement des cheveux, selon la revendication 1, contenant un pourcentage en poids de 0,1 à 10 d'au moins un acide gras C₁₀-C₂₄, calculé sur la constitution totale.

3. Utilisation d'un agent pour le traitement des cheveux, selon la revendication 1 ou 2, contenant un pourcentage en poids de 0,1 à 20 d'au moins une composition de la formule I, calculé sur la constitution totale.

4. Utilisation d'un agent pour le traitement des cheveux, selon une ou plusieurs des revendications 1 à 3, **caractérisée en ce que** les restes R¹ et R² représentent respectivement un reste oléylique, le reste R³ représente un groupement méthyle, le reste R⁴ représente un groupement -CH₂-CH₂-OH₂ ,et x et y représentent 0.

5. Utilisation d'un agent pour le traitement des cheveux, selon une ou plusieurs des revendications 1 à 3, **caractérisée en ce que** les restes R¹ et R² représentent respectivement un groupement alkyle C₁₂-C₁₂, le reste R³ représente un groupement méthyle et le reste R⁴ un groupement -CH₂-CH₂-O-[EO]_{y}H ,et x, y et z représentent 0.

6. Utilisation d'un agent pour le traitement des cheveux, selon une ou plusieurs des revendications 1 à 5, contenant de l'acide béhénique.

7. Utilisation d'un agent pour le traitement des cheveux, selon une ou plusieurs des revendications 1 à 6, contenant en outre au moins une composition sélectionnée dama le groupement méthoxy-1-propanol-2, éthoxyle-1-propanol-2, diéthylèneglycolmonoéthylèneéthyléther ou diéthylèneglycoléthyléther, dipropylèneglycolmonométhylèneéthyléther ou dipropylèneglycoléthyléther, alcool benzylique, oxyéthanol benzylique, alcool phényléthylique, phénoxyéthanol et/ou alcool cinnamique.

8. Utilisation d'un agent pour le traitement des cheveux, selon une ou plusieurs des revendications 1 à 7, contenant en outre au moins un sel d'ammonium quaternaire avec un ou deux groupements alkyle C₁₀-C₂₂.

9. Utilisation d'un agent pour le traitement des cheveux, selon une ou plusieurs des revendiçations 1 à 8, contenant en outre au moins un tensioactif amphotère resp. zwitterionique et/ou non ionique.

10. Utilisation d'un agent pour le traitement des cheveux, selon la revendication 9, contenant un polyglucoside d'alkyle C₃ à C ₂₀ comme tensioactif non ionique.
